# EUROPEAN PATENT SPECIFICATION

(11) **EP 3 525 756 B1**
(45) Date of publication and mention of the grant of the patent: **19.04.2023**
(21) Application number: 17781096.7
(22) Date of filing: 09.10.2017
(51) Int. Cl.: A61K 8/19, A61K 8/29, A61Q 1/02, A61Q 17/04, A61K 8/60

(54) **COSMETIC COMPOSITIONS COMPRISING ALKYLPOLYGLUCOSIDES AND MICROPIGMENTS**
KOSMETISCHE ZUSAMMENSETZUNGEN ENTAHLTEND ALKYLPOLYGLUCOSIDEN UND MIKROPIGMENT
COMPOSITIONS COSMÉTIQUES COMPRENANT DES ALKYLPOLYGLUCOSIDES ET DES MICROPIGMENTS

(30) Priority: 11.10.2016 EP 16193204
(43) Date of publication of application: 21.08.2019
(73) Proprietor: DSM IP Assets B.V., 6411 TE Heerlen (NL)
(72) Inventor: DESHAYES, Cyrille, 4303 Kaiseraugst (CH); HOELLER, Ulrich, 4303 Kaiseraugst (CH); JANSSEN, Anne, 4303 Kaiseraugst (CH)
(74) Representative: Berg, Katja
(86) International application number: PCT/EP2017/075593
(87) International publication number: WO 2018/069204

(56) References cited:
- EP-A1- 0 840 595
- EP-A1- 1 093 798
- EP-A1- 1 388 338
- WO-A2-2010/125543

## Description

The present invention relates to cosmetic compositions comprising at least one inorganic micropigment, characterized in that said cosmetic compositions are substantially free of any C₁₂₋₁₆ alkyl poly-glucoside.

Alkyl poly-glycosides (APGs) are a class of non-ionic surfactants widely used in a variety of household, cosmetic and industrial applications. APG's are derived from glucose of varying polymerization levels and fatty alcohols and have the generic formula CₙH₂₊ₙ O(C₆H₁₀O₅)ₓH, in which n is an integer selected in the range of 2 to 22 and x refers to the mean polymerization level of the glucoside moiety (mono-, di-, tri-, oligo-, and poly-glucoside). The raw materials for industrial manufacture thereof are typically corn derived glucose and plant derived fatty alcohols. The final products are typically complex mixtures of compounds with glucose moieties comprising the hydrophilic end and alkyl groups of variable length comprising the hydrophobic end.

It has now surprisingly been found that the use of C₈₋₁₆ alkyl poly-glucosides such as e.g. Plantacare^{®} UP 2000 in combination with inorganic micropigments in a cosmetic composition leads to an unwanted 'cottage-cheese' like appearance of said composition which is highly unwanted by the cosmetic industry. Furthermore, it has been found, that this effect can be avoided by the use of a C₈₋₁₀ alkyl poly-glucoside such as Green APG 0810.

Thus, in a first embodiment, the present invention relates to a cosmetic composition comprising at least one inorganic micropigment and a C₈₋₁₀ alkyl poly-glucoside, characterized in that said composition is substantially free of any C₁₂₋₁₆ alkyl poly-glucoside as defined in claim 1.

The term "substantially (i.e. essentially) free of any C₁₂₋₁₆ alkyl poly-glucoside" as defined herein means that the compositions of the present invention contain no appreciable amount of C₁₂₋₁₆ alkyl poly-glucosides, in particular no amounts which lead to the adverse effect in combination with the inorganic micropigment i.e., no more than 0.1 wt.-%, preferably no more than 0.05 wt.-%, most preferably no more than 0.01% such as in particular no more than 0.005 wt.-%, based on the total weight of the cosmetic compositions.

EP0840595 and EP1093378 disclose cosmetic compositions comprising at least one inorganic micropigment and a C8-C16 fatty alcohol polyglucoside.

The C₈₋₁₀ alkyl poly-glucosides generally exhibit a mean polymerisation level of the glucoside moiety ranging from 1 to 1.7, preferably from 1.1 to 1.6, most preferably from 1.1 to 1.4 such as in particular in the range of 1.1 to 1.3.

Further advantageous mean polymerisation level of the glucoside moiety range from 1.2 to 1.6 such as from 1.4 to 1.6. Additional advantageous mean polymerisation level of the glucoside moiety range from 1.2 to 1.7, respectively from 1.4 to 1.6.

Particularly advantageous C₈₋₁₀ alkyl poly-glucoside according to the invention consist essentially of caprylyl (C₈) and capryl (C₁₀) poly-glucosides. Preferably such caprylyl (C₈) and capryl (C₁₀) poly-glucosides furthermore exhibit a ratio (%/%, wherein all % are area-% determined by HPLC-MS) of caprylyl (C₈) mono-glucoside to capryl (C₁₀) mono-glucoside in the range of 3:1 to 1:3, preferably in the range of about 2:1 to 1:2, most preferably in the range of 1.5:1 to 1:1.5. Additionally, such C₈₋₁₀ alkyl poly-glucoside preferably contain no more than 3 wt.-%, more preferably no more than 2 wt.-%, most preferably no more than 1.5 wt.-% of C₁₂ alkyl mono-glucoside as determined by HPLC-MS and illustrated in the example. It is understood, that such alkyl poly-glucosides are free of any (i.e. contain no) higher (i.e. C₁₄₋₁₆) alkyl polyglucosides.

Thus, in an advantageous embodiment, the present invention also relates to the cosmetic composition according to present invention wherein the C₈₋₁₀alkyl poly-glucoside contains no more than 2 % of C₁₂ alkyl mono-glucoside. It is further preferred that such C₈₋₁₀alkyl polyglucosides do not contain any C₁₄₋₁₆ alkyl polyglucosides.

Furthermore, the C₈₋₁₀alkyl poly-glucoside according to the invention consisting essentially of caprylyl (C₈) and capryl (C₁₀) poly-glucosides contains advantageously at least 60%, preferably at least 65%, most preferably at least 70% of the respective mono-glucosides as e.g. determined by HPLC-MS.

It is furthermore preferred that the C₈₋₁₀alkyl poly-glucoside according to the present invention are substantially (i.e. essentially) free of any C₉ alkyl poly-glucosides, i.e. contain essentially no C₉ alkyl poly-glucosides. This means that the amount of any C₉ alkyl poly-glucosides in the C₈₋₁₀ alkyl poly-glucoside is less than 0.1 wt.-%, preferably less than 0.05 wt.-%, most preferably less than 0.01% such as in particular less than 0.005 wt.-%, based on the total weight of the C₈₋₁₀ alkyl poly-glucoside.

A particularly advantageous C₈₋₁₀alkyl poly-glucoside according to the present invention is made from glucose derived from corn and C₈ and C₁₀ fatty alcohols derived from coconut and palm kernel oils, which is e.g. sold as an aqueous dispersion under the tradename Green APG 0810 by Shanghai Fine Chemical.

The C₈₋₁₀alkyl poly-glucosides are advantageously incorporated into the cosmetic compositions according to the present invention in a total amount selected in the range of 0.001 to 5 wt.-%, preferably in the range of 0.01 to 2 wt.-%, most preferably in the range of 0.05 to 1.5 wt.-%, based on the total weight of the cosmetic composition.

The inorganic micropigments are advantageously incorporated into the cosmetic compositions according to the present invention in a total amount selected in the range of 0.1 to 40 wt.-%, preferably in the range of 1 to 30 wt.-%, based on the total weight of the cosmetic composition.

Particularly suitable inorganic micropigments in all embodiments of the present invention are metal powders, metal oxides or metal hydroxides conventionally used in cosmetic applications either as inorganic UV filter or as colouring agent. Exemplary inorganic micropigments according to the present invention encompass magnesium oxide, magnesium hydroxide, calcium oxide, calcium hydroxides, aluminum oxide, aluminum hydroxide, iron oxides (α-Fe₂O₃, γ-Fe₂O₃, Fe₃O₄, FeO), red iron oxide, yellow iron oxide, black iron oxide, iron hydroxides, titanium (di)oxides, zirconium oxides, chromium oxides, chromium hydroxides, manganese oxides, cobalt oxides, cerium oxides, nickel oxides and zinc oxides as well as composite oxides and composite hydroxides such as iron titanate, cobalt titanate and cobalt aluminate.

The inorganic micropigments according to the present invention may optionally be surface treated to, for example, make the particles more hydrophobic or more dispersible in a vehicle.

Particularly preferred inorganic micropigments according to the present invention are selected from the group consisting of titanium dioxides, zinc oxides and iron oxides, most preferably from titanium dioxide and iron oxide, as well as mixtures thereof.

In one particular advantageous embodiment the inorganic micropigment is an inorganic UV filter having a particle size which is principally useful for incorporation into sunscreen compositions such as in particular a titanium dioxide or zinc oxide UV filter.

These inorganic UV filters are preferably used in an amount (total) selected in the range of 0.1 to 20 wt.-%, preferably in the range of 0.5 to 10 wt.-%, more preferably in the range of 1 to 10 wt.-%, based on the total weight of the cosmetic composition.

Preferably, in all embodiments of the present invention, a titanium dioxide UV filter having an average primary particle size of about 2 nm to 100 nm, preferably of about 5 to 50 nm and a secondary particle size of about 0.05 to 50 µm, preferably of about 0.1 to1 µm is used.

The crystalline form of the titanium dioxide UV filter may be of any crystal or amorphous type. For example, titanium dioxide may be any type of amorphous, rutil, anastase, brookite or a mixture thereof.

In a preferred embodiment, the titanium dioxide UV filter used according to the present invention is coated with at least one coating such as in particular with aluminium hydroxide, a polyol, silica, a silicon oil such as methicone or dimethicone, or an alkyl silane. Such coatings are well known in the art. Commercially available single coated titanium dioxides suitable according to the invention are e.g. available as Uvinul^{®} TiO₂ (INCI: trimethoxycaprylylsilane and titanium dioxide ex BASF) or Eusolex^{®} T-Avo (INCI: Titanium dioxide, Silica ex Merck).

In a more particular embodiment of the invention, the titanium dioxide UV filter, however, is a double coated titanium dioxide as this leads to even better results. Such double coated titanium dioxides preferably have an inner coating selected from inorganic silica or aluminium hydroxide and an outer organic coating (referred to as double coated titanium dioxide). Preferably the outer organic coating is selected from silicone oils (e.g. simethicones, methicones, dimethicones, polysilicone-15), alkyl silanes, olefinic acids such as in particular stearic acid, polyols such as in particular glycerol or organophosphonic acids such as in particular cetyl phosphate.

In such double coated titanium dioxides the inner coating preferably consists of minimum 0.5 wt.-%, more preferably of 0.5-50 wt.-%, most preferably of 1-20 wt.-%, based on the weight of the non-coated titanium dioxide.

The outer coating layer preferably consists of minimum 0.25 wt.-%, preferably of 0.5-50 wt.-%, most preferably of 0.5-10 wt.-% of organic coating, based on the weight of the non-coated titanium dioxide.

Such double coated titanium dioxide nanoparticles can be prepared according to the state of the art or are commercially available as PARSOL^{®} TX (INCI: Titanium Dioxide, Silica, Dimethicone ex DSM Nutritional Products) or as UV-Titan X195 (coated with silica and treated with a silicone oil (i.e. methicone) ex Merck) or Tayca MT-100TV (Titanium Dioxide (and) Aluminum Hydroxide (and) Stearic Acid).

Other usual organic coatings can additionally be present in order to yield multiple coated (such as e.g. triple coated) titanium dioxide. The other coatings can be applied before, after or together with the second outer coating. Other additional coatings which can be used comprise organic coatings such as stearic acid, silicones (silane derivatives such as triethoxycaprylylsilane or siloxane derivatives such as methicone, dimethicone, simethicone).

In all embodiments of the present invention the titanium dioxide UV filter is most preferably a double coated titanium dioxide having an inner inorganic silica coating wherein the outer coating consists of simethicone, methicone, dimethicone (also known as polydimethylsiloxane), polysilicone-15, stearic acid, glycerol and mixtures thereof, in particular of methicone, dimethicone, stearic acid or mixtures thereof. Most preferably, the outer coating consists of methicone or dimethicone, in particular of dimethicone. The most preferred the titanium dioxide UV filter according to the invention are UV-Titan X195 by Huntsman and/or PARSOL^{®} TX by DSM Nutritional products which are titanium dioxide grades coated with silica (inner coating) and treated with a silicone oil such as in particular methicone (UV-Titan X195) or dimethicone (PARSOL^{®} TX) as outer coating. Most in particular PARSOL^{®} TX by DSM Nutritional products is used as titanium dioxide UV filter in the compositions according to the invention.

In another advantageous embodiment, the inorganic micropigment is a coloring agent conventionally used in decorative cosmetics such as make-up and/ or foundation compositions. Particularly suitable inorganic coloring agents according to the present invention are titanium dioxide, zirconium or cerium oxides, zinc, iron (black, yellow or red) or chromium oxides, manganese violet, ultramarine blue, chromium hydrate and ferric blue, or metal powders, such as aluminium powder or copper powder.

If present, the amount (total) of these inorganic coloring agent(s) is preferably selected in the range of 1 wt.-% to 40 wt.-%, preferably in the range of 2 wt.-% to 30 wt.-%, more preferably in the range of 5 wt.-% to 15 wt.-%, based on the total weight of the cosmetic composition.

The crystalline form of the iron and titanium dioxide coloring agent may be of any crystal or amorphous type suitable for that purpose. For example, titanium dioxide may be any type of amorphous, rutil, anastase, brookite or a mixture thereof. The particle shape of the iron oxide coloring agent may be of any acicular, spheroidal or cubic shape, as well as mixtures thereof.

Particularly preferred inorganic coloring agent according to the present invention are selected from the group consisting of iron oxide and titanium dioxide having a particle size ranging from about 0.001 to 150 µm, preferably from about 0.002 to 100 µm, more preferably from about 0.02 to 50 µm. Such inorganic coloring agents are well known to a person skilled in the art and e.g. commercially available under the tradename UNIPURE at Sensient.

In a preferred embodiment, the iron oxide and titanium dioxide coloring agents used according to the present invention are surface treated with an organic coating such as with an alkylsilane e.g. triethoxycaprylylsilane, with a silicone oil e.g. dimethicone or methicone, with an organo titanate, and/or with natural surface treatments e.g. polyhydroxystearic acid, stearoyl glutamic acid hydrogenated lecithin, jojoba esters and sodium glycerophosphate. Such coated inorganic coloring agents are well known to a person skilled in the art and e.g. commercially available under the tradename UNIPURE at Sensient, or from the product portfolio for coloring agents at KOBO, Merck.

Particularly suitable inorganic coloring agents for foundation and/ or make-up compositions according to the present invention include optionally surface treated titanium dioxides (rutile or anatase) listed in the Color Index under reference CI 77891 such as UNIPURE LC 981 AS-EM from Sensient. Further suitable inorganic coloring agents for foundation and/ or make-up compositions according to the present invention include black, yellow, red and brown iron oxides, optionally surface treated, listed in the Color Index under references Cl 77499, 77492 and 77491 such as Unipure RED LC381 from Sensient.

The cosmetic compositions according to the invention are intended for topical application, which is to be understood as the external application to keratinous substances, such as in particular the skin.

As the cosmetic compositions according to the invention are intended for topical application, they comprise a physiologically acceptable medium, that is to say a medium compatible with keratinous substances, such as the skin, mucous membranes, and keratinous fibers. In particular the physiologically acceptable medium is a cosmetically acceptable carrier.

The term `cosmetically acceptable carrier' as used herein refers to a physiologically acceptable medium which is compatible with keratinous substances. Suitable carriers are well known in the art and are selected based on the end-use application. Preferably, the carriers of the present invention are suitable for application to skin (e.g. in the form of creams, milks, lotions, masks, serums, hydrodispersions, foundations, creamgels, or gels etc.). Such carriers are well-known to one of ordinary skill in the art and can include one or more compatible liquid or solid filler diluent, excipient, adjuvant, additive or vehicle which are suitable for application to skin.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

Particularly suitable excipients, diluents, adjuvants additives for the compositions according to the present invention are cosmetic oils such as C12-15 alkyl benzoate, cetyl alcohol, cetearyl alcohol, capric/caprylic triglycerides, diisopropylsebacate, preservatives such as phenoxyethanol and ethlyhexylglycerin (Euxyl PE 9010 from Shülke& Mayr), parabens (Euxyl K 300 form Schülke&Mayr); thickening agents for the aqueous phase such as polysaccharide such as e.g. Xanthan Gum (Keltrol CGT from Kelco); biopolymers such as e.g. cellulose gum (Tylose CG 200 from SE Tylose); mineral thickeners such as e.g. magnesium aluminium silicate (Veegum from Vanderbilt), synthetic polymers such as e.g. carbomer (Carbopol 980 from Lubrizol), UV filters, fragrances as well as humectants such as e.g. glycerin and propylene glycol.

In accordance with the present invention, the compositions according to the invention may also comprise further cosmetically active ingredients conventionally used in cosmetic compositions. Exemplary active ingredients encompass skin lightening agents, UV filters, agents for the treatment of hyperpigmentation, agents for the prevention or reduction of inflammation, firming, moisturizing, soothing and/ or energizing agents as well as agents to improve elasticity and skin barrier.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

In a particularly preferred embodiment, the cosmetic compositions according to the present invention are sunscreen compositions for the protection of the skin against harmful UV-radiation or make-up and/ or foundation compositions for the provision of a uniform "base" skin color.

The compositions according to the present invention, in particular the sunscreen compositions, preferably comprise at least one further organic UV-filter substance (light screening agents) which is active in the UV-A and/or UV-B regions (absorbers), such UV-filter substances being watersoluble, fat-soluble or insoluble in commonly used cosmetic solvents.

Particularly advantageous UVA, UVB and/ or broadspectrum UV-filter substances to be incorporated into the cosmetic compositions according to the present invention are dibenzoylmethane derivatives such as e.g. butyl methoxydibenzoylmethane (PARSOL@ 1789); acrylates such as e.g. octocrylene (PARSOL@ 340); camphor derivatives such as e.g. 4-methyl benzylidene camphor (PARSOL@ 5000) or terephthalylidene dicamphor sulfonic acid (Mexoryl^{®} SX); cinnamate derivatives such as e.g. ethylhexyl methoxycinnamate (PARSOL@ MCX) or isoamyl methoxycinnamate; p aminobenzoic acid derivatives such as e.g. p aminobenzoic acid or 2-ethylhexyl p-dimethylaminobenzoate; benzophenones such as e.g. benzophenone-3, benzophenone-4, 2,2',4,4'-tetrahydroxy-benzophenone or 2,2'-dihydroxy-4,4'-dimethoxybenzophenone; esters of benzalmalonic acid such as e.g. di-(2-ethylhexyl) 4-methoxybenzalmalonate; organosiloxane compounds carrying chromophore groups such as e.g. polysilicone-15 (PARSOL@ SLX) or drometrizole trisiloxane (Mexoryl^{®} XL); imidazole derivatives such as e.g. 2-phenyl benzimidazole sulfonic acid and salts thereof such as e.g. its sodium- or potassium salts (PARSOL@ HS); salicylate derivatives such as e.g. ethylhexyl salicylate (PARSOL@ EHS, Neo Heliopan^{®} OS), isooctyl salicylate or homosalate (PARSOL@ HMS, Neo Heliopan^{®} HMS); triazine derivatives such as e.g. ethylhexyl triazone (Uvinul^{®} T-150), diethylhexyl butamido triazone (Uvasorb^{®} HEB), bis-ethylhexyloxyphenol methoxyphenyl triazine (Tinosorb^{®} S) or Tris-Biphenyl Triazine (2,4,6-Tris(biphenyl-4-yl)-1,3,5-triazin, Tinosorb^{®} A2B); Benzotriazole derivatives such as e.g. methylene bis-benzotriazolyl tetramethylbutylphenol (Tinosorb^{®} M); encapsulated UV-filters such as e.g. encapsulated ethylhexyl methoxycinnamate (Eusolex^{®} UV-pearls); amino substituted hydroxybenzophenones such as e.g. diethylamino hydroxybenzoyl hexyl benzoate (Aminobenzophenon, Uvinul^{®} A Plus); benzoxazol-derivatives such as e.g. 2,4-bis-[5-1(dimethylpropyl)benzoxazol-2-yl-(4-phenyl)-imino]-6-(2-ethylhexyl)-imino-1,3,5-triazin (Uvasorb^{®} K2A); phenylene-1,4-bis-benzimidazolsulfonic acids or salts thereof such as e.g. disodium phenyl dibenzimidazole tetrasulfonate (2,2-(1,4-phenylene)bis-(1H-benzimidazol-4,6-disulfonic acid, Neoheliopan^{®} AP); 1,1' (1,4-piperazinediyl)bis[1-[4-(diethylamino)-2-hydroxybenzoyl]phenyl]-methanone (CAS No. 919803-06-6); as well as Bis(butylbenzoate) diaminotriazine aminopropyltrisiloxane (CAS No. 207562-42-3).

Preferred UVB-filter substances to be incorporated into the cosmetic compositions according to the invention encompass polysilicone-15, phenylbenzimidazol sulfonic acid, octocrylene, ethylhexyl methoxycinnamate, ethyl hexylsalicylate, tris-biphenyl triazine and/ or homosalate.

Preferred broadband UV-filter substances to be incorporated into the cosmetic compositions according to the invention encompass unsymmetrical s triazine derivatives such as in particular bis-ethylhexyloxyphenol methoxyphenyl triazine, certain benzophenones such as e.g. 2-hydroxy-4-methoxy-benzophenon, and/ or methylene bis-benzotriazolyl tetramethylbutylphenol.

Preferred UVA-filter substances to be incorporated into the cosmetic compositions according to the invention encompass butyl methoxydibenzoylmethane, diethylamino hydroxybenzoyl hexyl benzoate, 2,4-bis-[5-1(dimethylpropyl)benzoxazol-2-yl-(4-phenyl)-imino]-6-(2-ethylhexyl)-imino-1,3,5-triazine and/ or disodium phenyl dibenzimidazole tetrasulfonate, in particular butyl methoxydibenzoylmethane and/ or diethylamino hydroxybenzoyl hexyl benzoate.

If the topical sunscreen emulsions comprise butyl methoxydibenzoylmethane, then they advantageously contain in addition at least one suitable photostabilizer for butyl methoxydibenzoylmethane. Besides specific UV-filters listed above which are known to a person skilled in the art to be able to photostabilize butyl methoxydibenzoylmethane, further exemplary photostabilizers encompass Polyester 8 (Polycrylene^{®}); Methoxycrylene (Solastay); diethylhexyl syringylidene malonate (Oxynex ST liquid); diethylhexyl naphthalate (Corapan TQ) as well as Benzotriazolyl Dodecyl p-Cresol (Tinogard^{®} TL) without being limited thereto. An overview on such photostabilizers is e.g. given in `SPF Boosters & Photostability of Ultraviolet Filters', HAPPI, October 2007, p. 77-83.

These photostabilizers are generally used in an amount of 0.05 to 10 wt. % with respect to the total weigh of the topical sunscreen emulsion.

Most preferably, in all embodiments of the present invention the cosmetic compositions additionally comprise at least methylene bis-benzotriazolyl as further UV filter substance, which is incorporated as an aqueous dispersion stabilized with a C₈₋₁₀ alkyl poly-glucoside.

The total amount of the additional UV-filter substances in the compositions according to the present invention is preferably selected in the range of 0.1 to 40 wt. %, more preferably in the range of 0.2 to 20 wt. % and most preferably in the range of 0.5 to 15 wt.-%, based on the total weight of the cosmetic composition.

The compositions according to the present invention may be in the form of a suspension or dispersion in solvents or fatty substances, or alternatively in the form of an emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

If the composition is an emulsion, such as in particular an O/W, W/O, Si/W, W/Si, O/W/O, W/O/W multiple or a pickering emulsion, then the amount of the oily phase present in such cosmetic emulsions is preferably at least 10 wt.-%, such as in the range of 10 to 60 wt.-%, preferably in the range of 15 to 50 wt.-%, most preferably in the range of 15 to 40 wt.-%, based on the total weight of the composition.

In one embodiment, the compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol^{®} A from DSM Nutritional Products Ltd.), diethanolamine cetyl phosphate (e.g. as Amphisol^{®} DEA from DSM Nutritional Products Ltd.), potassium cetyl phosphate (e.g. as Amphisol^{®} K from DSM Nutritional Products Ltd.), sodium cetearylsulfate, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the composition.

Particular suitable O/W emulsifiers to be used in the compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate.

A particular suitable O/W emulsifier to be used in the compositions according to the invention is potassium cetyl phosphate e.g. commercially available as Amphisol^{®} K at DSM Nutritional Products Ltd Kaiseraugst.

Another particular suitable class of O/W emulsifiers are non-ionic self-emulsifying systems derived from olive oil e.g. known as (INCI Name) cetearyl olivate and sorbitan olivate (chemical composition: sorbitan ester and cetearyl ester of olive oil fatty acids) sold under the tradename OLIVEM 1000.

In one particular embodiment, the invention relates to compositions with all the definitions and preferences given herein in the form of O/W emulsions comprising an oily phase dispersed in an aqueous phase in the presence of an O/W emulsifier wherein the O/W emulsifier is potassium cetyl phosphate. The amount of oily phase in such O/W emulsions is preferably at least 10 wt.-%, more preferably in the range of 10 to 60 wt.-%, most preferably in the range of 15 to 50 wt.-%, such as in the range of 15 to 40 wt.-%.

The compositions according to the invention in general have a pH in the range of 3 to 10, preferably a pH in the range of 4 to 8 and most preferably a pH in the range of 4 to 7.5. The pH can easily be adjusted as desired with suitable acids, such as e.g. citric acid, or bases, such as sodium hydroxide (e.g. as aqueous solution), triethanolamine (TEA Care), tromethamine (Trizma Base) and Aminomethyl Propanol (AMP-Ultra PC 2000), according to standard methods in the art.

The following examples are provided to further illustrate the compositions and effects of the present invention. These examples are illustrative only and are not intended to limit the scope of the invention in any way.

### Example

### 1. Analytics

The respective samples have been dissolved in a mixture of tetrahydrofurane / water (50/50), approx. 1 mg / ml, and were analysed by HPLC mass spectrometry using a reversed-phase YMC Pro C4 column with a water/acetonitrile gradient with 0.1% methanesulfonic acid (5 -> 90 % acetonitrile over 15 min). Detection was performed on an Agilent 6130 single MSD operating in ES positive mode. TIC and EIC were used to determine the relative distribution of the compounds of interest. The relative distribution of the alkyl monoglucosides is outlined in table 1. All % are area-%.

**Table 1: Relative distribution of the alkyl monoglucosides**

| | ***Relative amount* [%]** | |
|---|---|---|
| ***Sample*** | ***Alkyl (8-16) Glucoside**** | ***C8-C10 polyglucoside°*** |
| C₈ monoglucoside | 20.8 | 44.1 |
| C₁₀ monoglucoside | 16.6 | 54.7 |
| C₁₂ monoglucoside | 41.5 | 1.2 |
| C₁₄ monoglucoside | 20.1 | n.d. |
| C₁₆ monoglucoside | 0.9 | n.d. |

| | | |
|---|---|---|
| *Commercially available as Plantacare UP 2000 at Cognis; approx. absolute amount as determined by HPLC-MS of C₈/C₁₀/ C₁₂/C₁₄/C₁₆ alkyl monoglucoside ~ 77% (area %) °Commercially available as Green APG 0810 at Shanghai Fine Chemicals; approx. absolute amount as determined by HPLC-MS of C₈/C₁₀ alkyl monoglucoside ~ 78% (area %) n.d.: not detected | | |

### 2. Formulation

To 19.6 g of Sun Ozon Sonnenspray SPF 30 containing titanium dioxide (nano) (Formulation I) either 0.4g of C8-C16 poly-glucoside (Plantacare UP 2000) or 0.4 g of C8-C10 poly-glucoside (APG 0810) has been added. The results are presented in table 2.

**Table 2: Results Formulation I**

| | ***Alkyl (8-16) Glucoside**** | ***C8-C10 polyglucoside°*** |
|---|---|---|
| After 5 min | TiO₂ agglomerate | fluid cream, conform |
| After 30min | oil exudation & agglomerate | fluid cream, conform |

Furthermore, the formulations as outlined in table 2 have been prepared and analysed via microscopy as well as visually to assess the acceptability of the product form. As can be retrieved, only the samples prepared a C₈₋₁₀ poly-glucoside according to the present invention (Green APG 0810) lead to an acceptable product form while the use of a C₈₋₁₆ poly-glucoside (Plantacare UP 2000) lead to a significant agglomeration or the titanium dioxide and even to phase separation of the emulsion.

**Table 3: Formulations II**

| **INCI *(Tradename)*** | **Ref 1** | **Inv 1** | **Ref 2** | **Inv 2** | **Ref 3** | **Inv 3** |
|---|---|---|---|---|---|---|
| Octocrylene *(Parsol^{®} 340)* | 10.00 | 10.00 | 10.00 | 10.00 | 10.00 | 10.00 |
| C12-15 Alkyl Benzoate *(Finsolv^{®} TN)* | 5.00 | 5.00 | 5.00 | 5.00 | 5.00 | 5.00 |
| Dicaprylyl Ether *(Cetiol^{®} OE)* | 2.00 | 2.00 | 2.00 | 2.00 | 2.00 | 2.00 |
| Homosalate *(Parsol^{®} HMS)* | 8.00 | 8.00 | 8.00 | 8.00 | 8.00 | 8.00 |
| Ethylhexyl Salicylate *(Parsol EHS)* | 5.00 | 5.00 | 5.00 | 5.00 | 5.00 | 5.00 |
| Titanium Dioxide (and) Silica *(Eusolex^{®} T-AVO)* | 4.50 | 4.50 | | | | |
| Titanium Dioxide (and) Aluminum Hydroxide (and) Stearic Acid *(Tayca MT-100TV)* | | | 4.50 | 4.50 | | |
| Titanium Dioxide (and) Silica (and) Dimethicone *(Parsol^{®} TX)* | | | | | 4.50 | 4.50 |
| Butyl Methoxydibenzoylmethane *(Parsol^{®} 1789)* | 4.00 | 4.00 | 4.00 | 4.00 | 4.00 | 4.00 |
| VP/Hexadecene Copolymer (Antaron V-216) | 1.00 | 1.00 | 1.00 | 1.00 | 1.00 | 1.00 |
| Aqua *(WATER DEM)* | Ad 100 | Ad 100 | Ad 100 | Ad 100 | Ad 100 | Ad 100 |
| Glycerin | 6.00 | 6.00 | 6.00 | 6.00 | 6.00 | 6.00 |
| Carbomer *(Carbopol^{®} Ultrez 30)* | 0.10 | 0.10 | 0.10 | 0.10 | 0.10 | 0.10 |
| Xanthan Gum | 0.15 | 0.15 | 0.15 | 0.15 | 0.15 | 0.15 |
| *(Keltrol^{®} CG-T)* | | | | | | |
| Acrylates/C10-30 Alkyl Acrylate Crosspolymer *(Pemulen^{®} TR-2)* | 0.30 | 0.30 | 0.30 | 0.30 | 0.30 | 0.30 |
| Sodium-EDTA *(Na-EDTA)* | 0.10 | 0.10 | 0.10 | 0.10 | 0.10 | 0.10 |
| Phenoxyethanol (and) Ethylhexylglycerin (Euxyl^{®} PE 9010) | 1.00 | 1.00 | 1.00 | 1.00 | 1.00 | 1.00 |
| Alcohol *(Ethanol)* | 8.00 | 8.00 | 8.00 | 8.00 | 8.00 | 8.00 |
| Decylglucoside (Plantacare^{®} 200 UP) | 2.00 | | 2.00 | | 2.00 | |
| Decylglucoside (Green APG 0810) | | 2.00 | | 2.00 | | 2.00 |
| pH | 6.11 | 6.19 | 6.25 | 6.26 | 5.92 | 5.94 |
| Agglomeration | strong | few | strong | some | strong | few |
| Phase separation | Yes | No | No | No | No | No |
| Acceptable product form | No | Yes | No | Yes | No | Yes |

## Claims

1. A cosmetic composition comprising at least one inorganic micropigment and a C₈₋₁₀ alkyl poly-glucoside, **characterized in that** the composition is substantially free of any C₁₂₋₁₆ alkyl poly-glucoside and the term substantially free means an amount of no more than 0.1 wt.-%, based on the total weight of the cosmetic compositions.

2. The cosmetic composition according to claim 1, **characterized in that** the composition contains no more than 0.05 wt.-%, preferably no more than 0.01%, most preferably no more than 0.005 wt.-% of C₁₂₋₁₆ alkyl poly-glucosides, based on the total weight of the cosmetic compositions

3. The cosmetic composition according to claim 1, **characterized in that** the amount of the C₈₋₁₀ alkyl poly-glucoside is selected in the range of 0.001 to 5 wt.-%, preferably in the range of 0.01 to 2 wt.-%, most preferably in the range of 0.05 to 1.5 wt.-%, based on the total weight of the cosmetic composition.

4. The cosmetic composition according to any one of claims 1 to 3, **characterized in that** the amount of the at least one inorganic micropigment is selected in the range of 0.1 to 40 wt.-%, preferably in the range of 1 to 30 wt.-%, based on the total weight of the cosmetic composition.

5. The cosmetic composition according to any one of claims 1 to 4, **characterized in that** the C₈₋₁₀ alkyl poly-glucoside consists essentially of caprylyl/ capryl poly-glucosides.

6. The cosmetic composition according to claim 5, **characterized in that** the ratio of caprylyl mono-glucoside to capryl mono-glucoside in the caprylyl/ capryl poly-glucosides is selected in the range of 3:1 to 1:3.

7. The cosmetic composition according to any one of claims 1 to 6, **characterized in that** the C₈₋₁₀ alkyl poly-glucoside is made from glucose derived from corn and fatty alcohols derived from coconut and palm kernel oils.

8. The cosmetic composition according to any one of claims1 to 7, **characterized in that** the inorganic micropigment is an inorganic UV filter or a coloring agent.

9. The cosmetic composition according to claim 8, **characterized in that** the inorganic micropigment is a titanium dioxide coated with at least one coating, preferably with aluminium hydroxide, a polyol, silica, a silicon oil or an alkyl silane.

10. The cosmetic composition according to claim 9, **characterized in that** the titanium dioxide is a double coated titanium dioxide having an inner aluminium hydroxide or inorganic silica coating and an outer organic coating selected from the group consisting of simethicone, methicone, dimethicone, polysilicone-15, cetyl phosphate, stearic acid and mixtures thereof.

11. The cosmetic composition according to claim 8, **characterized in that** the coloring agent is an iron oxide or a titanium dioxide having a particle size selected in the range of .001 to 150 µm, preferably in the range of 0.002 to 100 µm, more preferably in the range of 0.02 to 50 µm.

12. The cosmetic composition according to any one of claims 1 to 11, **characterized in that** the composition comprises at least one further organic UV filter substance.

13. The composition according to claim 12, **characterized in that** the at least one further organic UV-filter substance encompasses methylene bis-benzotriazolyl tetramethylbutylphenol.

14. The cosmetic composition according to any one of claims 1 to 13, **characterized in that** the cosmetic composition is an oil-in-water (O/W) emulsion comprising an oily phase dispersed in an aqueous phase.

15. The cosmetic composition according to any one of claims 1 to 14, **characterized in that** the composition is a sunscreen, or a make-up/ foundation composition.

## Patentansprüche

1. Kosmetische Zusammensetzung, umfassend mindestens ein anorganisches Mikropigment und ein C₈₋₁₀-Alkylpolyglucosid, **dadurch gekennzeichnet, dass** die Zusammensetzung weitgehend frei von jeglichem C₁₂₋₁₆-Alkylpolyglucosid ist und der Begriff weitgehend frei eine Menge von nicht mehr als 0,1 Gew.-%, bezogen auf das Gesamtgewicht der kosmetischen Zusammensetzung, bedeutet.

2. Kosmetische Zusammensetzung nach Anspruch 1, **dadurch gekennzeichnet, dass** die Zusammensetzung nicht mehr als 0,05 Gew.-%, vorzugsweise nicht mehr als 0,01 Gew.-%, ganz besonders bevorzugt nicht mehr als 0,005 Gew.-%, C₁₂₋₁₆-Alkylpolyglucoside, bezogen auf das Gesamtgewicht der kosmetischen Zusammensetzung, enthält.

3. Kosmetische Zusammensetzung nach Anspruch 1, **dadurch gekennzeichnet, dass** die Menge des C₈₋₁₀-Alkylpolyglucosids im Bereich von 0,001 bis 5 Gew.-%, vorzugsweise im Bereich von 0,01 bis 2 Gew.-%, ganz besonders bevorzugt im Bereich von 0,05 bis 1,5 Gew.-%, bezogen auf das Gesamtgewicht der kosmetischen Zusammensetzung, ausgewählt ist.

4. Kosmetische Zusammensetzung nach einem der Ansprüche 1 bis 3, **dadurch gekennzeichnet, dass** die Menge des mindestens einen anorganischen Mikropigments im Bereich von 0,1 bis 40 Gew.-%, vorzugsweise im Bereich von 1 bis 30 Gew.-%, bezogen auf das Gesamtgewicht der kosmetischen Zusammensetzung, ausgewählt ist.

5. Kosmetische Zusammensetzung nach einem der Ansprüche 1 bis 4, **dadurch gekennzeichnet, dass** das C₈₋₁₀-Alkylpolyglucosid im Wesentlichen aus Caprylyl-/Caprylpolyglucosiden besteht.

6. Kosmetische Zusammensetzung nach Anspruch 5, **dadurch gekennzeichnet, dass** das Verhältnis von Caprylylmonoglucosid zu Caprylmonoglucosid in den Caprylyl-/Caprylpolyglucosiden im Bereich von 3:1 bis 1:3 ausgewählt ist.

7. Kosmetische Zusammensetzung nach einem der Ansprüche 1 bis 6, **dadurch gekennzeichnet, dass** das C₈₋₁₀-Alkylpolyglucosid aus Glucose, die aus Mais gewonnen wurde, und Fettalkoholen, die aus Kokosnuss- und Palmkernöl gewonnen wurden, besteht.

8. Kosmetische Zusammensetzung nach einem der Ansprüche 1 bis 7, **dadurch gekennzeichnet, dass** es sich bei dem anorganischen Mikropigment um einen anorganischen UV-Filter oder ein Farbmittel handelt.

9. Kosmetische Zusammensetzung nach Anspruch 8, **dadurch gekennzeichnet, dass** es sich bei dem anorganischen Mikropigment um Titandioxid, das mit mindestens einer Beschichtung, vorzugsweise mit Aluminiumhydroxid, einem Polyol, Siliciumdioxid, einem Silikonöl oder einem Alkylsilan, beschichtet ist, handelt.

10. Kosmetische Zusammensetzung nach Anspruch 9, **dadurch gekennzeichnet, dass** es sich bei dem Titandioxid um ein doppelt beschichtetes Titandioxid mit einer inneren Beschichtung aus Aluminiumhydroxid oder anorganischem Siliciumdioxid und einer äußeren organischen Beschichtung, die aus der Gruppe bestehend aus Simethicon, Methicon, Dimethicon, Polysilikon-15, Cetylphosphat, Stearinsäure und Mischungen davon ausgewählt ist, handelt.

11. Kosmetische Zusammensetzung nach Anspruch 8, **dadurch gekennzeichnet, dass** es sich bei dem Farbmittel um ein Eisenoxid oder ein Titandioxid mit einer Teilchengröße, die im Bereich von 0,001 bis 150 um, vorzugsweise im Bereich von 0,002 bis 100 um, weiter bevorzugt im Bereich von 0,02 bis 50 um, ausgewählt ist, handelt.

12. Kosmetische Zusammensetzung nach einem der Ansprüche 1 bis 11, **dadurch gekennzeichnet, dass** die Zusammensetzung mindestens eine weitere organische UV-Filtersubstanz umfasst.

13. Zusammensetzung nach Anspruch 12, **dadurch gekennzeichnet, dass** die mindestens eine weitere organische UV-Filtersubstanz Methylenbisbenzotriazolyltetramethylbutylphenol umfasst.

14. Kosmetische Zusammensetzung nach einem der Ansprüche 1 bis 13, **dadurch gekennzeichnet, dass** es sich bei der kosmetischen Zusammensetzung um eine Öl-in-Wasser(O/W)-Emulsion handelt, die eine in einer wässrigen Phase dispergierte ölige Phase umfasst.

15. Kosmetische Zusammensetzung nach einem der Ansprüche 1 bis 14, **dadurch gekennzeichnet, dass** es sich bei der Zusammensetzung um ein Sonnenschutzmittel oder eine Make-up-/Grundierungszusammensetzung handelt.

## Revendications

1. Composition cosmétique comprenant au moins un micropigment inorganique et un (alkyle en C₈₋₁₀)polyglucoside, **caractérisée en ce que** la composition est sensiblement exempte d'un quelconque (alkyle en C₁₂₋₁₆) polyglucoside et les termes « sensiblement exempte » signifient une quantité de pas plus de 0,1 % en poids, sur la base du poids total des compositions cosmétiques.

2. Composition cosmétique selon la revendication 1, **caractérisée en ce que** la composition ne contient pas plus de 0,05 % en poids, préférablement pas plus de 0,01 %, le plus préférablement pas plus de 0,005 % en poids de (alkyle en C₁₂₋₁₆) polyglucosides, sur la base du poids total des compositions cosmétiques.

3. Composition cosmétique selon la revendication 1, **caractérisée en ce que** la quantité du (alkyle en C₈₋₁₀)polyglucoside est choisie dans la plage de 0,001 à 5 % en poids, préférablement dans la plage de 0,01 à 2 % en poids, le plus préférablement dans la plage de 0,05 à 1,5 % en poids, sur la base du poids total de la composition cosmétique.

4. Composition cosmétique selon l'une quelconque des revendications 1 à 3, **caractérisée en ce que** la quantité de l'au moins un micropigment inorganique est choisie dans la plage de 0,1 à 40 % en poids, préférablement dans la plage de 1 à 30 % en poids, sur la base du poids total de la composition cosmétique.

5. Composition cosmétique selon l'une quelconque des revendications 1 à 4, **caractérisée en ce que** le (alkyle en C₈₋₁₀) polyglucoside est essentiellement constitué de caprylyl/capryl-polyglucosides.

6. Composition cosmétique selon la revendication 5, **caractérisée en ce que** le rapport de caprylyl-monoglucoside sur capryl-monoglucoside dans les caprylyl/capryl-polyglucosides est dans la plage de 3 : 1 à 1 : 3.

7. Composition cosmétique selon l'une quelconque des revendications 1 à 6, **caractérisée en ce que** le (alkyle en C₈₋₁₀) polyglucoside est composé de glucose issu de maïs et d'acides gras issus d'huiles de noix de coco et de palmiste.

8. Composition cosmétique selon l'une quelconque des revendications 1 à 7, **caractérisée en ce que** le micropigment inorganique est un filtre UV inorganique ou un agent colorant.

9. Composition cosmétique selon la revendication 8, **caractérisée en ce que** le micropigment inorganique est un dioxyde de titane revêtu par au moins un revêtement, préférablement par de l'hydroxyde d'aluminium, un polyol, une silice, une huile de silicone ou un alkylsilane.

10. Composition cosmétique selon la revendication 9, **caractérisée en ce que** le dioxyde de titane est un dioxyde de titane doublement revêtu possédant un revêtement interne d'hydroxyde d'aluminium ou de silice inorganique et un revêtement organique externe choisi dans le groupe constitué par une siméthicone, une méthicone, une diméthicone, une polysilicone-15, le phosphate de cétyle, l'acide stéarique et des mélanges correspondants.

11. Composition cosmétique selon la revendication 8, **caractérisée en ce que** l'agent colorant est un oxyde de fer ou un dioxyde de titane possédant une taille de particule choisie dans la plage de 0,001 à 150 um, préférablement dans la plage de 0,002 à 100 um, plus préférablement dans la plage de 0,02 à 50 µm.

12. Composition cosmétique selon l'une quelconque des revendications 1 à 11, **caractérisée en ce que** la composition comprend au moins une substance de filtre UV organique supplémentaire.

13. Composition selon la revendication 12, **caractérisée en ce que** l'au moins une substance de filtre UV organique supplémentaire comprend du méthylène bis-benzotriazolyl-tétraméthylbutylphénol.

14. Composition cosmétique selon l'une quelconque des revendications 1 à 13, **caractérisée en ce que** la composition cosmétique est une émulsion huile-dans-eau (O/W) comprenant une phase huileuse dispersée dans une phase aqueuse.

15. Composition cosmétique selon l'une quelconque des revendications 1 à 14, **caractérisée en ce que** la composition est un écran solaire, ou une composition de maquillage/de fond de teint.
